Europäisches Patentamt

European Patent Office

Office européen des brevets

(11) Veröffentlichungsnummer: **0 045 459**

**A1**

(12) **EUROPÄISCHE PATENTANMELDUNG**

(21) Anmeldenummer: **81105854.4**

(22) Anmeldetag: **24.07.81**

(51) Int. Cl.³: **C 07 C 85/145**
**C 07 C 87/50**

(30) Priorität: **01.08.80 DE 3029265**

(43) Veröffentlichungstag der Anmeldung:
**10.02.82 Patentblatt 82/6**

(84) Benannte Vertragsstaaten:
**DE FR GB IT**

(71) Anmelder: **HOECHST AKTIENGESELLSCHAFT**
**Postfach 80 03 20**
**D-6230 Frankfurt/Main 80(DE)**

(72) Erfinder: **Bodenbenner, Kurt, Dr.**
**Hermann-Kaiser-Strasse 4**
**D-6200 Wiesbaden(DE)**

(72) Erfinder: **Durruti-Cubria, Manuel, Dr.**
**Stroofstrasse 4**
**D-6230 Frankfurt am Main 80(DE)**

(72) Erfinder: **Woernle, Rolf, Dr.**
**Martin-Wohmann-Strasse 27**
**D-6238 Hofheim am Taunus(DE)**

(54) Verfahren zur kontinuierlichen Herstellung von Diphenylbasen.

(57) Die Umlagerung von Hydrazoaromaten zu Diphenylbasen in nich wassermischbaren aromatischen Lösemitteln mit Mineralsäuren kann kontinuierlich erfolgen, wenn man die Lösung der Hydrazoverbindung in dem aromatischen Lösemittel mit einer solchen Menge an Mineralsäure versetzt, daß eine förderbare Suspension erhalten wird. Vorteilhaft wird nach der Umlagerung das aromatische Lösemittel aus der Reaktionsmischung abgetrennt, insbesondere in einem kontinuierlichen Verfahren, und anschließend das Salz ebenfalls kontinuierlich abgetrennt. Das Filtrat kann nach Aufstärken mit konzentrierter Mineralsäure in den Prozess zurückgeführt werden. Es können somit alle Hilfsstoffe vollständig in den Prozeß zurückgeführt werden.

## Verfahren zur kontinuierlichen Herstellung von Diphenylbasen

. Diphenylbasen, insbesondere die in 3,3'-Stellung durch Chlor, Methyl oder Methoxy substituierten 4,4'-Diaminodiphenyle, sind wichtige Ausgangsmaterialien für Disazoverbindungen, wobei diese Stoffe einerseits direkt als Bis-Diazokomponente dienen und andererseits auch als Bis-N-acetoacetylverbindung als Kupplungskomponente eingesetzt werden.

Diphenylbasen werden üblicherweise durch sogenannte Benzidin-Umlagerung aus den entsprechenden Hydrazoaromaten hergestellt, die ihrerseits durch Reduktion der entsprechenden Nitrobenzole gewonnen werden. Diese Umlagerung wird durch Mineralsäuren katalysiert.

Bei der Durchführung der Umlagerung in mit Wasser mischbaren Lösemitteln fallen als Nebenprodukte die entsprechenden Diphenyline (2,4'-Diaminobiphenyle) an, und zwar in um so größerer Menge, je weniger polar das verwendete Lösemittel ist. Es wird deshalb üblicherweise eine Lösung des Hydrazoaromaten in einem aromatischen Lösemittel der Umlagerung mit Mineralsäure unterworfen. Hierbei findet die Umlagerung in der wäßrigen Phase statt und das sich abscheidende Salz der Diphenylbase bildet mit dem aromatischen Lösemittel und der Mineralsäure eine Suspension, die mit fortschreitender Umsetzung so viskos wird, daß sie weder rührbar noch pumpfähig ist. Bei der weiteren Umsetzung ist also der Stoffaustausch auf die Diffusion angewiesen, was zu langen Reaktionszeiten führt. Diese haben wiederum zur Folge, daß störende Nebenreaktionen, zum Beispiel die Disproportionierung des Hydrazoaromaten, stärker hervortreten. Die dadurch gebildeten Nebenprodukte vermindern nicht nur direkt die Ausbeute, sondern auch indirekt durch den erhöhten Reinigungsaufwand.

Die zur Umlagerung verwendete Säuremenge wird üblicherweise so niedrig wie möglich gehalten, um den Aufwand
bei der erforderlichen Aufarbeitung der Abwässer gering
zu halten.

Es wurde nun gefunden, daß die Umlagerung kontinuierlich
durchgeführt werden kann, wenn man die Mineralsäure
in einem so großen Überschuß einsetzt, daß eine förderbare Suspension erhalten wird.

Gegenstand der Erfindung ist deshalb ein Verfahren zur
Herstellung der Mineralsäure-Salze von Diphenylbasen
durch Umlagerung der entsprechenden Hydrazoaromaten
in nicht-wassermischbaren aromatischen Lösemitteln
mit Mineralsäuren, das dadurch gekennzeichnet ist,
daß man die Lösung der Hydrazoverbindung in dem aromatischen Lösemittel kontinuierlich mit einer solchen
Menge an Mineralsäure versetzt, daß eine förderbare
Suspension erhalten wird.

Im folgenden werden bevorzugte Ausgestaltungen der
Erfindung näher erläutert:

Als Lösemittel für die Hydrazoverbindung kommen alle
inerten aromatischen Stoffe in Betracht, die ein ausreichendes Lösevermögen zeigen. Von technischer Bedeutung
sind Toluol, Xylol und Lösemittelgemische, beispielsweise
das unter der Bezeichnung "Solventnaphtha" handelsübliche
Gemisch aus m-Xylol und Ethylbenzol. Vorteilhaft verwendet man Lösungen des Hydrazoaromaten, wie sie bei
der Reduktion der entsprechenden Nitroverbindung in
einem geeigneten nicht-wassermischbaren aromatischen
Lösemittel anfallen, insbesondere in einem der genannten
Kohlenwasserstoffe.

Die Mineralsäuren werden zweckmäßig als wäßrige Säuren
eingesetzt, beispielsweise als 20 bis 80 Gew.-%ige Schwefelsäure oder als 10 bis 30 Gew.-%ige Salzsäure.
Die Säure wird zweckmäßig in einem etwa 8 bis 16-fachen,
bevorzugt 10 bis 14-fachen Verhältnis, bezogen auf die
zur Salzbildung erforderliche Menge, eingesetzt.

Die Reaktionstemperatur richtet sich nach der Reaktivität
der Hydrazoverbindung und liegt üblicherweise im Bereich
von etwa 20 bis 50°C.

Größe und Art des Reaktionsraumes hängen von der
erforderlichen Verweilzeit des Reaktionsgutes ab, die
ihrerseits an den eingesetzten Hydrazoaromaten und die
Reaktionstemperatur gebunden ist. Sind Verweilzeiten
von 1 bis 3 Stunden erforderlich, arbeitet man vorteilhaft in einer Kesselkaskade, die aus höchstens 5 gerührten
Einzelkesseln besteht. Prinzipiell sind auch andere
Reaktionsapparate möglich, insbesondere dann, wenn
in Folge hoher Reaktionsgeschwindigkeiten geringere
Verweilzeiten möglich sind.

Besonders vorteilhaft ist es, das aromatische Lösemittel
nach der Umlagerung aus der Reaktionsmischung abzutrennen.
Diese Abtrennung erfolgt zweckmäßig durch Destillation
unter Vakuum oder bei Normaldruck. Sofern das Lösemittel
gut wasserdampfflüchtig ist und die dabei eintretende
Verdünnung der Reaktionsmischung nicht stört, kann die
Abtrennung auch mit Dampf erfolgen. Azeotrop übergehendes
Wasser, das bei Verwendung von Salzsäure als Mineralsäure Chlorwasserstoff enthält, wird zweckmäßig zurückgeführt. In diesem Falle wird außerdem gasförmiger
Chlorwasserstoff frei, der in der zurückzuführenden Säure
absorbiert werden kann.

Besonders vorteilhaft ist eine kontinuierliche Abtrennung
des Lösemittels, wobei man beispielsweise das Reaktionsgemisch aus der Umlagerung direkt in einen Verdampfer
leitet. Das Abdampfen des Lösemittels kann in einem Dünnschichtverdampfer, aber auch in hintereinander geschalteten Kesseln durchgeführt werden, da für diese bevorzugte Ausführungsform nicht die Art der Apparatur, sondern der kontinuierliche Verfahrensablauf die entscheidende Rolle spielt. Die Siedetemperatur kann durch Einstellen des Druckes zwischen etwa 10 mbar und 1 bar (abs.) so gehalten werden, daß Zersetzungserscheinungen vermieden werden.

Die lösemittelfreie Suspension ist problemlos filtrierbar. Eine besonders zweckmäßige Ausführungsform der Erfindung besteht deshalb darin, daß nach Abtrennung des Lösemittels das Salz kontinuierlich abgetrennt wird. Eine besondere Auswahl unter möglichen Apparaten ist nicht erforderlich. Kontinuierlich arbeitende Filter, beispielsweise Bandfilter, sind ebenso einsetzbar wie Zentrifugen.

Der Rückstand stellt das rohe, feuchte Salz der Diphenylbase dar, das anschließend erforderlichenfalls in bekannter Weise gereinigt werden kann.

Das Filtrat wird zweckmäßig nach Aufstärken mit frischer konzentrierter Mineralsäure in den Prozeß zurückgeführt.

Das erfindungsgemäße Verfahren zeigt - insbesondere in seinen bevorzugten Ausführungsformen - eine Reihe von Vorteilen: Mit einem Minimum an Operationen können die Salze der Diphenylbasen in hoher Ausbeute und bei hohem Durchsatz erhalten werden, wobei die eingesetzten Hilfsstoffe vollständig in den Prozeß zurückgeführt werden.

An Hand der folgenden Beispiele und der Figuren werden besonders zweckmäßige Ausgestaltungen der Erfindung näher erläutert. Prozentangaben beziehen sich hierbei auf das Gewicht, sofern nichts anderes angegeben ist.

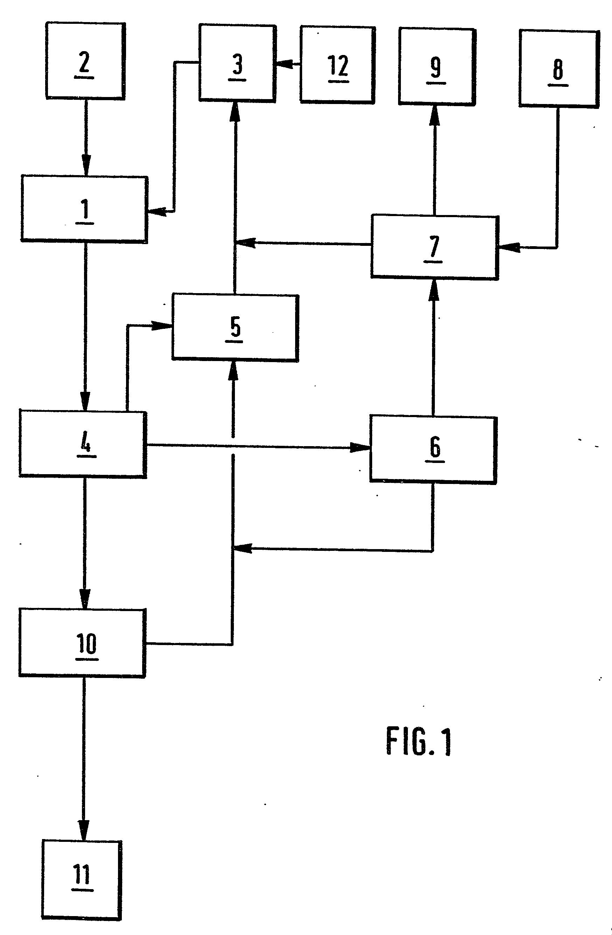

Beispiel 1 (Figur 1)

In eine Kesselkaskade (1), die aus 3 gerührten Kesseln mit je 2 m$^3$ Inhalt besteht, wird pro Stunde aus dem Vorratsgefäß (2) eine Lösung von 330 kg 2,2'-Dichlorhydrazobenzol, gelöst in 900 kg Toluol, eindosiert. Gleichzeitig werden aus dem Vorratsgefäß (3) stündlich 3900 kg 30 %ige Salzsäure mengengeregelt eingegeben. Die Temperatur in den Kesseln (1) wird auf 35°C gehalten, was mit Hilfe eines temperaturgeregelten Kühlwasserstroms erreicht wird. Die aus dem dritten Kessel (standgeregelt) ablaufende Suspension wird in einen Dünnschichtverdampfer (4) geleitet, in dem das Toluol und eine relativ geringe Menge azeotrop übergehende Salzsäure abdestilliert werden. Gleichzeitig abgetriebener gasförmiger Chlorwasserstoff wird in eine Absorptionskolonne (5) geleitet. In der Kondensationsanlage (6) wird das Toluol von der in den Prozeß zurückgehenden wäßrigen Phase getrennt, im Wäscher (7) mit Wasser aus dem Gefäß (8) gewaschen und in das Vorratsgefäß (9) gefördert. Es wird erneut zur Herstellung der Einsatzlösung des 2,2'-Dichlorhydrazobenzols verwendet. Das toluolfreie Sumpfprodukt gelangt über ein Filterband (10), auf dem das rohe Salz von überschüssiger Säure getrennt wird, in das Austraggefäß (11). Die abgetrennte Säure wird über die Absorptionskolonne (5) und das Vorratsgefäß (3) in die Kesselkaskade (1) zurückgeleitet, nachdem durch Zugabe von konzentrierter Salzsäure aus dem Vorratsgefäß (12) die Ursprungskonzentration von 30 % Chlorwasserstoff eingestellt und die Gesamtmenge auf 3900 kg pro Stunde ergänzt wurde.

Der Filterkuchen kann durch mehrmaliges Waschen mit Wasser oder Salzsäure vorgereinigt und anschließend durch Umkristallisieren aus Salzsäure gereinigt werden. Eine Reinigung ist auch über die freie Base möglich.

Die Ausbeute an rohem Salz beträgt über 95 % der Theorie.

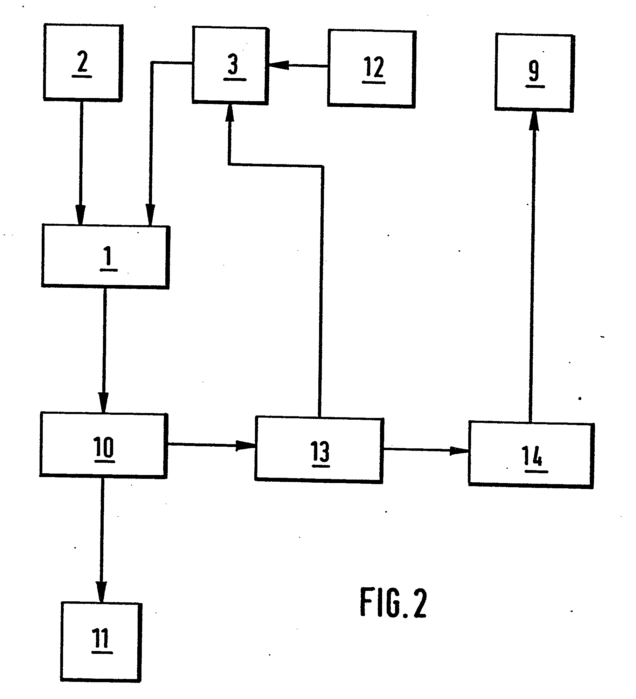

Beispiel 2 (Figur 2)

In einer Apparatur, die der in Beispiel 1 verwendeten entspricht, bei der jedoch der Dünnschichtverdampfer (4), die Absorptionskolonne (5), die Kondensationsanlage (6) und der Wäscher (7) durch eine Scheideflasche (13) und eine Rektifikationskolonne (14) ersetzt sind, setzt man pro Stunde eine Lösung von 330 kg 2,2'-Dichlorhydrazobenzol, gelöst in 1000 kg Xylol, aus dem Vorratsgefäß (2) mit 3400 kg 50 %iger Schwefelsäure aus dem Vorratsgefäß (3) in der Kesselkaskade (1) kontinuierlich um. Hierbei wird die Temperatur in (1) im Bereich von 30 - 45°C gehalten.

Die aus der Kesselkaskade (1) ablaufende Suspension wird über das Filterband (10) filtriert. Das Filtrat, das aus Xylol und etwa 48 %iger Schwefelsäure besteht, wird in der Scheideflasche (13) getrennt. Die Schwefelsäure wird in das Vorratsgefäß (3) zurückgeleitet, in dem durch Zugabe von frischer Schwefelsäure aus dem Vorratsgefäß (12) Konzentration und Menge der Einsatzsäure wieder eingestellt werden. Das abgetrennt Xylol wird in der Rektifikationskolonne (14) gereinigt und dem Vorratgefäß (9) zugeführt. Es wird zur Herstellung der Lösung des 2,2'-Dichlorhydrazobenzols wieder eingesetzt.

Der Filterkuchen wird wie in Beispiel 1 auf dem Filterband (10) von überschüssiger Säure befreit und in das Austragsgefäß (11) gefördert. Die Reinigung kann entsprechend Beispiel 1 erfolgen.

PATENTANSPRÜCHE:

1. Verfahren zur Herstellung der Mineralsäure-Salze von Diphenylbasen durch Umlagerung der entsprechenden Hydrazoaromaten in nicht-wassermischbaren aromatischen Lösemitteln mit Mineralsäuren, dadurch gekennzeichnet, daß man die Lösung der Hydrazoverbindung in dem aromatischen Lösemittel kontinuierlich mit einer solchen Menge an Mineralsäure versetzt, daß eine förderbare Suspension erhalten wird.

2. Verfahren nach Anspruch 1, dadurch gekennzeichnet, daß nach der Umlagerung das aromatische Lösemittel aus der Reaktionsmischung abgetrennt wird.

3. Verfahren nach Anspruch 2, dadurch gekennzeichnet, daß die Abtrennung des Lösemittels kontinuierlich erfolgt.

4. Verfahren nach Anspruch 3, dadurch gekennzeichnet, daß nach Abtrennung des Lösemittels das Salz kontinuierlich abgetrennt wird.

5. Verfahren nach Anspruch 4, dadurch gekennzeichnet, daß das Filtrat nach Aufstärken mit konzentrierter Mineralsäure in den Prozeß zurückgeführt wird.

6. Verfahren nach Anspruch 1 bis 5, dadurch gekennzeichnet, daß das aromatische Lösemittel ein Kohlenwasserstoff ist.

7. Verfahren nach Anspruch 1 bis 6, dadurch gekennzeichnet, daß als Mineralsäure 20 bis 80 gewichtsprozentige Schwefelsäure oder 10 bis 30 gewichtsprozentige Salzsäure eingesetzt werden.

8. Verfahren nach Anspruch 1 bis 7, dadurch gekennzeichnet, daß das 8- bis 16-fache der Menge, die zur Salzbildung erforderlich ist, an Mineralsäure eingesetzt wird.

9. Verfahren nach Anspruch 1 bis 8, dadurch gekennzeichnet, daß das 10- bis 14-fache der Menge, die zur Salzbildung erforderlich ist, an Mineralsäure eingesetzt wird.

10. Verfahren nach Anspruch 1 bis 9, dadurch gekennzeichnet, daß die Umsetzung bei 20 bis 50°C durchgeführt wird.

1/2

FIG. 1

0045459

2/2

FIG.2

0045459

Europäisches Patentamt

**EUROPÄISCHER RECHERCHENBERICHT**

Nummer der Anmeldung

EP 81 10 5854.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - C - 1 030 833 (K. DÜRKES)<br>* Spalte 4, Zeilen 5 bis 9 *<br>-- | 1 |
| A | Houben-Weyl "Methoden der organischen Chemie", 4. Auflage, Band XI/1<br>1957, GEORG THIEME VERLAG, Stuttgart<br>* Seiten 839 bis 846 *<br>--- | |
| A | US - A - 1 718 373 (NATIONAL ANILINE & CHEMICAL CO.)<br>-- | |
| A | Chemical Abstracts Band 57, Nr. 5,<br>3. September 1962<br>Columbus, Ohio, USA<br>L.B. SHAGALOV et al. "Automation of the production of benzidine"<br>Abstract Nr. 5755d<br>& Vestn. Tekhn. i Ekon. Inform. Nauchn.-Issled., Nr. 4, 1961, Seiten 30 bis 35<br>---- | |

**KLASSIFIKATION DER ANMELDUNG (Int. Cl.³)**

C 07 C 85/145
C 07 C 87/50

**RECHERCHIERTE SACHGEBIETE (Int. Cl.³)**

C 07 C 85/145
C 07 C 87/50

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| | Der vorliegende Recherchenbericht wurde für alle Patentansprüche erstellt. |
|---|---|

| Recherchenort | Abschlußdatum der Recherche | Prüfer |
|---|---|---|
| Berlin | 27-10-1981 | PHILLIPS |

EPA form 1503.1 06.78